# EUROPÄISCHE PATENTSCHRIFT

(11) **EP 3 284 351 B1**
(45) Veröffentlichungstag und Bekanntmachung des Hinweises auf die Patenterteilung: **27.02.2019**
(21) Anmeldenummer: 16185056.5
(22) Anmeldetag: 20.08.2016
(51) Int. Cl.: A23L 3/26, A23B 9/06

(54) **VERFAHREN ZUM PASTEURISIEREN UND/ODER STERILISIEREN VON PARTIKELFÖRMIGEM GUT**
METHOD OF PASTEURIZING AND/OR STERILISING PARTICULATE MATERIAL
PROCEDE DE PASTEURISATION ET/OU DE STERILISATION DE MATIERE PARTICULAIRE

(43) Veröffentlichungstag der Anmeldung: 21.02.2018
(73) Patentinhaber: Bühler AG, 9240 Uzwil (CH)
(72) Erfinder: Nicols Meneses, 9200 Gossau (CH); Alasdair Currie, London N7 8QB (GB); Thomas Scheiwiller, 9524 Zuzwil (CH); Martin Hersche, 9000 St. Gallen (CH); Nikolaus Schönenberger, 9100 Herisau (CH)
(74) Vertreter: Hepp Wenger Ryffel AG

(56) Entgegenhaltungen:
- EP-A1- 1 625 859
- EP-B1- 0 705 531
- DATABASE WPI Week 200348 Thomson Scientific, London, GB; AN 2003-508327 XP002766847, -& JP 2003 000213 A (ONO K) 7. Januar 2003 (2003-01-07)
- DATABASE WPI Week 200258 Thomson Scientific, London, GB; AN 2002-541061 XP002766848, -& JP 2002 085031 A (JAPAN SCI&TECHNOLOGY AGENCY) 26. März 2002 (2002-03-26)

## Beschreibung

Die vorliegende Erfindung betrifft Verfahren zum Pasteurisieren und/oder Sterilisieren von partikelförmigem Gut mit Hilfe eines Elektronenstrahls.

Als partikelförmig werden hier und im Folgenden unter anderem aus Körnern und/oder Flocken bestehende Güter bezeichnet, wobei die Partikel beispielsweise eine kugelförmige, plattenförmige oder kantige Form haben können. Es kann sich auch um gemahlene Partikel handeln. Durch die Pasteurisierung und/oder Sterilisierung können beispielsweise Mikroorganismen zumindest grösstenteils abgetötet oder unschädlich gemacht werden. Insbesondere kann eine Reduktion von schädigenden Mikroorganismen um mindestens fünf Grössenordnungen erreicht werden.

Eine Vorrichtung zum Pasteurisieren und/oder Sterilisieren von partikelförmigem Gut ist beispielsweise aus der EP 1 080 623 B1 bekannt. Diese Vorrichtung enthält Vibrationsförderer, mit denen Saatgut zu einem transparenten Vorhang vereinzelt werden kann. Dieser Vorhang wird dann durch ein Elektronenfeld geführt, das von einem Elektronenbeschleuniger erzeugt wird und beispielsweise eine Sterilisierung des Saatguts bewirken kann.

Aus der US 5,801,387 A ist eine weitere Vorrichtung zum Pasteurisieren und/oder Sterilisieren von partikelförmigem Gut bekannt. In der dortigen erfindungsgemässen Ausführung wird ein partikelförmiges Gut mit einem Vibrationsförderer in einen horizontalen Luftstrom eindosiert und dann einem Elektronenstrahl ausgesetzt. Anschliessend wird mit Hilfe einer Vakuumpumpe und eines Filters eine Klassierung vollzogen.

Weiterhin offenbart die DE 10 2012 209 434 A1 eine Vorrichtung, die ein rieselfähiges Produkt mit Hilfe einer Vibrationsfördereinrichtung und einer rotierenden Bürstenwalze vereinzelt und in Rotationen versetzt. Anschliessend passieren die Partikel frei fallend ein Elektronenfeld.

In der EP 0 513 135 B1 ist eine Vorrichtung offenbart, mit der Saatgut mittels Zellradschleusen in einen vertikalen Fallschacht eingeleitet wird, wo es im senkrechten Fall von Elektronenstrahlen beaufschlagt wird.

Aus der EP 0 705 531 B1 ist eine weitere Vorrichtung bekannt, die das Saatgut mittels einer nicht näher beschriebenen Dosiereinrichtung in eine Prozesskammer eingeleitet wird, in der es senkrecht durch einen Elektronenstrahl fällt.

Die in US 6,486,481 B1 offenbarte Vorrichtung enthält einen Rütteltisch, auf dem ein polymeres Material bewegt und einem Elektronenstrahl ausgesetzt wird. Dies erfolgt jedoch nicht zur Pasteurisierung oder Sterilisierung, sondern zur Reduktion des Molekulargewichts des polymeren Materials.

Es ist eine Aufgabe der vorliegenden Erfindung, die aus dem Stand der Technik bekannten Nachteile zu überwinden. Insbesondere sollen Verfahren bereitgestellt werden, mit denen partikelförmiges Gut effektiv, zuverlässig und möglichst einfach, schnell und kostengünstig pasteurisiert und/oder sterilisiert werden kann.

Diese und weitere Aufgaben werden gelöst durch das erfindungsgemässe Verfahren zum Pasteurisieren und/oder Sterilisieren von partikelförmigem Gut. Es enthält die folgenden Schritte:
a) Erzeugen eines Elektronenstrahls,
b) Pasteurisieren und/oder Sterilisieren des Guts mittels des Elektronenstrahls in einer Behandlungszone.

Die Elektronen des Elektronenstrahls weisen erfindungsgemäss eine Energie auf, die im Bereich von 80 keV bis 300 keV, bevorzugt von 140 keV bis 280 keV, besonders bevorzugt von 180 keV bis 260 keV liegt. Geringere Elektronenenergien würden keine ausreichende Pasteurisierung und/oder Sterilisierung erzeugen. Durch höhere Elektronenenergien liessen sich keine wesentlich höheren Grade der Pasteurisierung und/oder Sterilisierung erreichen.

Weiterhin erfindungsgemäss liegt die Elektronenstromdichte in der Behandlungszone erfindungsgemäss im Bereich von 10¹⁵ s⁻¹·cm⁻² bis 2,77·10¹⁵ s⁻¹·cm⁻². In diesem Bereich wird eine ausreichende Pasteurisierung und/oder Sterilisierung erreicht. Ebenfalls erfindungsgemäss wird das Gut dem Elektronenstrahl für eine Behandlungszeit ausgesetzt, die im Bereich von 5 ms bis 25 ms liegt. Für eine ausreichende Pasteurisierung und/oder Sterilisierung ist eine gewisse minimale Behandlungszeit nötig. Zu lange Behandlungszeiten haben keinen nennenswert erhöhten Grad der Pasteurisierung und/oder Sterilisierung gezeigt.

Bei dem Gut kann es sich um ein Lebensmittel handeln, wie beispielsweise Getreide wie etwa Soja, Frühstückscerealien, Snacks, Nüsse wie etwa getrocknete Kokosnüsse, Mandeln, Erdnussbutter, Kakaobohnen, Schokolade, Schokoladenflüssigkeit, Schokoladenpulver, Schokoladenchips, Kakaoprodukte, Hülsenfrüchte, Kaffee, Samen wie etwa Kürbissamen, Gewürze (wie beispielsweise Kurkuma, insbesondere in Scheiben), Teemischungen, getrocknete Früchte, Pistazien, trockene Proteinprodukte, Bäckereiprodukte, Zucker, Kartoffelprodukte, Teigwaren, Babynahrung, getrocknete Eiprodukte, Sojaprodukte wie beispielsweise Sojabohnen, Verdickungsmittel, Hefen, Hefeextrakte, Gelatine oder Enzyme handeln.

Alternativ kann das Gut auch ein Tiernahrungsmittel sein, wie beispielsweise Pellets, Futter für Wiederkäuer, Geflügel, Wassertiere (insbesondere Fische) oder Haustiere, oder Mischfutter.

Es ist jedoch ebenso denkbar und liegt im Rahmen der Erfindung, dass das Gut beispielsweise ein Kunststoff wie etwa PET ist, beispielsweise in Form von Flocken oder Pellets.

Mit Vorteil wird das Gut mittels des Elektronenstrahls einer Strahlendosis ausgesetzt, die im Bereich von 1 kGy bis 45 kGy, bevorzugt von 8 kGy bis 30kGy, besonders bevorzugt von 10 kGy bis 16 kGy liegt.

Vorteilhafterweise wird das Gut vor der Behandlung im Schritt b) vereinzelt. Durch diese Vereinzelung kann sichergestellt werden, dass jedes einzelne Korn des Guts vom Elektronenstrahl erfasst und somit pasteurisiert und/oder sterilisiert wird. Eine Vereinzelung kann beispielsweise mit Hilfe von Vibrationsflächen erreicht werden, die zu Vibrationen angeregt werden, die optional über eine oder mehrere Rinnen verfügen. Alternativ oder zusätzlich kann eine Vereinzelung durch eine Rutschfläche erreicht werden, auf welcher das Gut herunterrutscht.

Ebenfalls mit Vorteil fällt das Gut frei durch die Behandlungszone. Das Gut wird dabei als "frei fallend" bezeichnet, wenn die Flugbahnen der einzelnen Partikel des Guts allein durch ihre Geschwindigkeit, die auf sie einwirkende Schwerkraft und gegebenenfalls ein Prozessgas, von dem das Gut umgeben ist, bestimmt werden. Insbesondere rutschen die Partikel des Guts nicht auf einer Fläche durch die Behandlungszone. Beim freien Fall ist die Geschwindigkeit unabhängig vom Durchsatz, so dass beispielsweise Durchsätze im Bereich 100 kg/h bis 1000 kg/h bei der gleichen Geschwindigkeit erreicht werden können.

Für viele Güter, insbesondere für eine Vielzahl von Gewürzen, hat es sich als vorteilhaft erwiesen, wenn sich das Gut mit einer Geschwindigkeit durch die Behandlungszone bewegt, die im Bereich von 1 m/s bis 5 m/s, bevorzugt von 2 m/s bis 4 m/s, besonders bevorzugt von 2 m/s bis 3 m/s liegt. Je höher die Geschwindigkeit des Guts ist, desto grösser ist der erreichbare Durchsatz. Andererseits dürfen die Geschwindigkeiten aber auch nicht zu gross gewählt werden, damit das Gut ausreichend lange im Elektronenstrahl verbleibt, um pasteurisiert und/oder sterilisiert zu werden.

Im Folgenden wird die Erfindung anhand zweier Ausführungsbeispiels und Zeichnungen näher erläutert. Dabei zeigen
- Figur 1:: eine schematische Darstellung zu einem ersten erfindungsgemässen Verfahren;
- Figur 2:: eine schematische Darstellung zu einem zweiten erfindungsgemässen Verfahren.

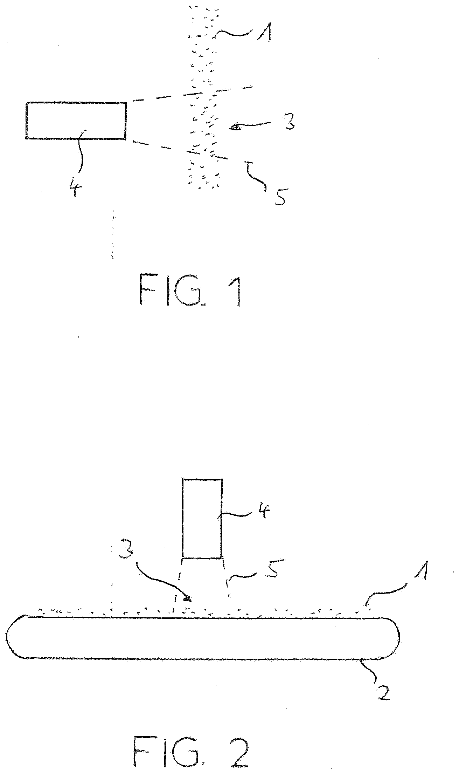

Im ersten Ausführungsbeispiel gemäss Figur 1 schematisch fällt ein partikelförmiges, vereinzeltes Gut 1, wie beispielsweise ein Gewürz, Pistazien oder Mandeln, mit einer zunehmenden Geschwindigkeit im Bereich von 1 m/s bis 5 m/s frei durch eine Behandlungszone 3 hindurch. Dort wird es mittels eines von einer Elektronenquelle 4 erzeugten Elektronenstrahls pasteurisiert und/oder sterilisiert. Der Elektronenstrahl enthält Elektronen einer Energie im Bereich von 80 keV bis 300 keV und hat in der Behandlungszone 3 eine mittlere Elektronenstromdichte im Bereich von 10¹⁵ s⁻¹·cm⁻² bis 2,77·10¹⁵ s⁻¹·cm⁻². Dieser Behandlung wird das Gut 1 für eine Behandlungszeit im Bereich von 5 ms bis 25 ms unterzogen, wodurch es einer Strahlendosis im Bereich von 1 kGy bis 45 kGy ausgesetzt wird.

Figur 2 zeigt schematisch ein zweites Ausführungsbeispiel. Darin wird ein vereinzeltes partikelförmiges Gut 1 auf ein Förderband 2 dosiert. Das Förderband 2 transportiert das Gut 1 in einer Behandlungszone 3 unter einer Elektronenquelle 4 her. Diese erzeugt in der Behandlungszone 3 einen Elektronenstrahl mit Elektronen einer Energie im Bereich von 80 keV bis 300 keV und einer mittleren Elektronenstromdichte im Bereich von 10¹⁵ s⁻¹·cm⁻² bis 2,77·10¹⁵ s⁻¹·cm⁻². Dieser Behandlung wird das Gut 1 für eine Behandlungszeit im Bereich von 5 ms bis 25 ms unterzogen, wodurch es einer Strahlendosis im Bereich von 1 kGy bis 45 kGy ausgesetzt wird.

Mit diesen Verfahren kann das partikelförmige Gut 1 effektiv und zuverlässig, aber dennoch möglichst einfach, schnell und kostengünstig pasteurisiert und/oder sterilisiert werden.

## Patentansprüche

1. Verfahren zum Pasteurisieren und/oder Sterilisieren von partikelförmigem Gut (1), enthaltend die folgenden Schritte:
a) Erzeugen eines Elektronenstrahls (5),
b) Pasteurisieren und/oder Sterilisieren des Guts (1) mittels des Elektronenstrahls (5) in einer Behandlungszone (3), wobei
- die Elektronen des Elektronenstrahls (5) eine Energie aufweisen, die im Bereich von 80 keV bis 300 keV, bevorzugt von 140 keV bis 280 keV, besonders bevorzugt von 180 keV bis 260 keV liegt,
- der Elektronenstrahl (5) in der Behandlungszone (3) eine mittlere Elektronenstromdichte aufweist, die im Bereich von 10¹⁵ s⁻¹·cm⁻² bis 2,77·10¹⁵ s⁻¹·cm⁻² liegt,
- das Gut (1) dem Elektronenstrahl (5) für eine Behandlungszeit ausgesetzt wird, die im Bereich von 5 ms bis 25 ms liegt.

2. Verfahren gemäss Anspruch 1,
wobei das Gut (1) mittels des Elektronenstrahls (5) einer Strahlendosis ausgesetzt wird, die im Bereich von 1 kGy bis 45 kGy, bevorzugt von 8 kGy bis 30 kGy, besonders bevorzugt von 10 kGy bis 16 kGy liegt.

3. Verfahren gemäss einem der vorangehenden Ansprüche,
wobei das Gut (1) vor Schritt b) vereinzelt wird.

4. Verfahren gemäss einem der vorangehenden Ansprüche,
wobei das Gut (1) frei durch Behandlungszone (3) fällt.

5. Verfahren gemäss einem der vorangehenden Ansprüche,
wobei sich das Gut (1) mit einer Geschwindigkeit (v) durch die Behandlungszone (3) bewegt, die im Bereich von 1 m/s bis 5 m/s, bevorzugt von 2 m/s bis 4 m/s, besonders bevorzugt von 2 m/s bis 3 m/s liegt.

6. Verfahren gemäss einem der vorangehenden Ansprüche,
wobei das Gut (1) aus der Gruppe ausgewählt ist, die besteht aus:
- Lebensmitteln, wie beispielsweise Getreide wie etwa Soja, Frühstückscerealien, Snacks, Nüssen wie etwa getrockneten Kokosnüssen, Mandeln, Erdnussbutter, Kakaobohnen, Schokolade, Schokoladenflüssigkeit, Schokoladenpulver, Schokoladenchips, Kakaoprodukten, Hülsenfrüchten, Kaffee, Samen wie etwa Kürbissamen, Gewürzen (wie beispielsweise Kurkuma, insbesondere in Scheiben), Teemischungen, getrockneten Früchten, Pistazien, trockenen Proteinprodukten, Bäckereiprodukten, Zucker, Kartoffelprodukten, Teigwaren, Babynahrung, getrockneten Eiprodukten, Sojaprodukten wie etwa Sojabohnen, Verdickungsmitteln, Hefen, Hefeextrakten, Gelatine oder Enzymen;
- Tiernahrungsmitteln, wie beispielsweise Pellets, Futter für Wiederkäuer, Geflügel, Wassertiere (insbesondere Fische) oder Haustiere, oder Mischfutter;
- Kunststoffen wie etwa PET, beispielsweise in Form von Flocken oder Pellets.

## Claims

1. A method for pasteurising and/or sterilising particulate material (1), containing the following steps:
a) producing an electron beam (5),
b) pasteurising and/or sterilising the material (1) by means of the electron beam (5) in a treatment zone (3), wherein
- the electrons of the electron beam (5) have an energy that lies in the range of from 80 keV to 300 keV, preferably from 140 keV to 280 keV, particularly preferably from 180 keV to 260 keV,
- the electron beam (5) in the treatment zone (3) has a mean electron current density that lies in the range of from 10¹⁵s⁻¹·cm⁻² to 2.77·10¹⁵s⁻¹·cm⁻²,
- the material (1) is exposed to the electron beam (5) for a treatment time that lies in the range of from 5 ms to 25 ms.

2. The method according to claim 1,
wherein the material (1), by means of the electron beam (5), is exposed to a dose of radiation that lies in the range of from 1 kGy to 45 kGy, preferably from 8 kGy to 30 kGy, particularly preferably from 10 kGy to 16 kGy.

3. The method according to either one of the preceding claims,
wherein the material (1) is separated prior to step b).

4. The method according to any one of the preceding claims,
wherein the material (1) falls freely through the treatment zone (3).

5. The method according to any one of the preceding claims,
wherein the material (1) is moved through the treatment zone (3) at a speed (v) that lies in the range of from 1 m/s to 5 m/s, preferably from 2 m/s to 4 m/s, particularly preferably from 2 m/s to 3 m/s.

6. The method according to any one of the preceding claims,
wherein the material (1) is selected from the group consisting of:
- foodstuffs, such as grains, for example soya, breakfast cereals, snacks, nuts, such as dried coconut, almonds, peanut butter, cocoa beans, chocolate, liquid chocolate, chocolate powder, chocolate chips, cocoa products, pulses, coffee, seeds, for example pumpkin seeds, spices (such as turmeric, in particular sliced), tea mixtures, dried fruits, pistachios, dried protein products, bakery products, sugar, potato products, pasta products, baby food, dried egg products, soya products, for example soya beans, thickeners, yeasts, yeast extracts, gelatines or enzymes;
- animal feed, such as pellets, food for ruminants, birds, aquatic species (in particular fish) or domestic pets, or compound feed;
- plastics, such as PET, for example in the form of flakes or pellets.

## Revendications

1. Procédé de pasteurisation et/ou de stérilisation d'un produit (1) sous forme particulaire, comprenant les étapes suivantes :
a) génération d'un rayonnement électronique (5),
b) pasteurisation et/ou stérilisation du produit (1) au moyen du rayonnement électronique (5) dans une zone de traitement (3), où
- les électrons du rayonnement électronique (5) présentent une énergie qui se situe dans la plage de 80 keV à 300 keV, de préférence, de 140 keV à 280 keV, de manière particulièrement préférée de 180 keV à 260 keV,
- le rayonnement électronique (5) présente dans la zone de traitement (3) une densité de courant électronique moyenne qui se situe dans la plage de 10¹⁵ s⁻¹ · cm⁻² à 2,77 · 10¹⁵ s⁻¹ · cm⁻²,
- le produit (1) est soumis au rayonnement électronique (5) pour une durée de traitement qui se situe dans la plage de 5 ms à 25 ms.

2. Procédé selon la revendication 1,
dans lequel le produit (1) est soumis à une dose de rayonnement au moyen du rayonnement électronique (5) qui se situe dans la plage de 1 kGy à 45 kGy, de préférence, de 8 kGy à 30 kGy, de manière particulièrement préférée, de 10 kGy à 16 kGy.

3. Procédé selon l'une des revendications précédentes,
dans lequel le produit (1) est isolé avant l'étape b).

4. Procédé selon l'une des revendications précédentes,
dans lequel le produit (1) tombe librement à travers la zone de traitement (3).

5. Procédé selon l'une des revendications précédentes,
dans lequel le produit (1) se déplace à travers la zone de traitement (3) avec une vitesse (v) qui se situe dans la plage de 1 m/s à 5 m/s, de préférence, de 2 m/s à 4 m/s, de manière particulièrement préférée de 2 m/s à 3 m/s.

6. Procédé selon l'une des revendications précédentes,
dans lequel le produit (1) est choisi dans le groupe qui est constitué de :
- produits alimentaires, comme, par exemple, de céréales comme par exemple, le soja, les céréales pour le petit déjeuner, d'en-cas, de noix, comme, par exemple, des noix de coco séchées, d'amandes, de beurre de cacahuètes, de fèves de cacao, de chocolat, d'un liquide à base de chocolat, de poudre de chocolat, de chips de chocolat, de produits à base de cacao, de fruits à coques, de café, de graines comme, par exemple, des graines de courges, d'épices (comme par exemple, de curcuma, notamment en tranches), de mélanges de thés, de fruits secs, de pistaches, de produits protéinés secs, de produits de boulangerie, de sucre, de produits à base de pomme de terre, de pâtes, de nourriture infantile, de produits à base d'oeuf secs, de produits à base de soja, comme, par exemple, des graines de soja, de produits épaississants, de levures, d'extraits de levure, de gélatine ou d'enzymes ;
- de produits d'alimentation animale, comme, par exemple, des boulettes, de la nourriture pour des ruminants, pour la volaille, pour les animaux aquatiques (notamment, les poissons), ou les pour animaux domestiques, ou des aliments composés ;
- de produits synthétiques, comme, par exemple, le PET, par exemple, sous la forme de flocons ou de boulettes.
